(19)

Europäisches
Patentamt
European
Patent Office
Office européen
des brevets

(11)     **EP 1 903 787 A2**

(12)                                     **EUROPEAN PATENT APPLICATION**

(43) Date of publication:
     **26.03.2008 Bulletin 2008/13**

(51) Int Cl.:
     ***H04N 5/325*** (2006.01)

(21) Application number: **07116213.5**

(22) Date of filing: **12.09.2007**

(84) Designated Contracting States:
     **AT BE BG CH CY CZ DE DK EE ES FI FR GB GR
     HU IE IS IT LI LT LU LV MC MT NL PL PT RO SE
     SI SK TR**
     Designated Extension States:
     **AL BA HR MK YU**

(30) Priority: **21.09.2006 JP 2006255574**

(71) Applicant: **Konica Minolta Medical & Graphic, Inc.
     Hino-shi,
     Tokyo 191-8511 (JP)**

(72) Inventors:
     • **Kido, Kazuhiro
       Konica Minolta Medical & Graphic, Inc.
       Tokyo 192-8505 (JP)**
     • **Oishi, Atsushi
       Konica Minolta Medical & Graphic, Inc.
       Tokyo 191-8511 (JP)**
     • **Umeda, Toshikazu
       Konica Minolta Medical & Graphic, Inc.
       Tokyo 192-8505 (JP)**

(74) Representative: **Henkel, Feiler & Hänzel
     Patentanwälte
     Maximiliansplatz 21
     80333 München (DE)**

(54)     **Image processing device and image processing method**

(57)     To improve accuracy of diagnosis by making image restoration possible for obtaining high definition images in accordance with positions for examination.

An image processing device includes: an image data input section for inputting image data obtained by using a detector to read a radiation ray applied from a radiation source and passed through a subject; a position input section for inputting a position for examination on the subject; a memory section for storing in advance image restoration parameters which correspond respectively to positions for examination on the subject; and a processing section for generating a restored image based on an image restoration parameter read from the memory section which corresponds to the position for examination on the subject that is input by the position input section and the image data that is input from the image data input section.

**EP 1 903 787 A2**

## Description

This application is based on Japanese Patent Application NO. 2006-255574 filed on September 21, 2006 with Japan Patent Office, the entire content of which is hereby incorporated by reference.

## FIELD OF THE INVENTION

The present invention relates to an image processing device and an image processing method. In particular, the present invention relates to an image processing device and image processing method in which restoration processing is performed for a radiographic image.

## BACKGROUND OF THE INVENTION

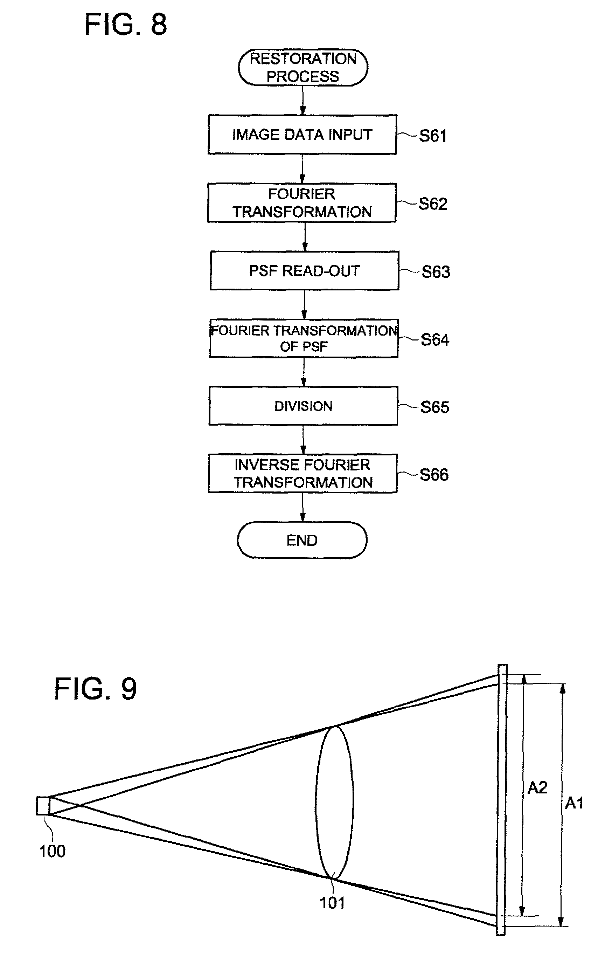

In the past, fine images have been desired for early discovery of focus of disease, diseased areas and lesions in radiographic image radiography. As a result, detection performance of the radiation detector used in radiography has been increased, but the scattered radiation that is generated in the subject is also detected and this causes the image to deteriorate. In particular, as shown in Fig. 9, the radiation source 100 can only have a fixed area, and thus when radiation beams are irradiated on the object 101, a displacement occurs between the projection region A1 resulting from the radiation ray emitted from the upper end of the radiation source 100 and the projection region A2 resulting from the radiation ray emitted from the lower end of the radiation source 100, and because there is a disparity in the luminance distribution of the edge portion, a blurred image was obtained.

In recent years, it has been proposed that the point spread function (referred to as PSF hereinafter) that is computed based on the radiographing conditions is used as a image restoration parameter in restoration processing to thereby restore blurred images and thus high definition images in which blurring is reduced are obtained (See Japanese Patent Publication No. 2509181 and Japanese Patent Publication No. 3423828 for example).

In Japanese Patent Publication No. 2509181, a radiographic image radiographing system is described in which PSF which shows the scattered X-ray distribution on the detector surface for the X-rays irradiated onto the subject is corrected in accordance with radiographing conditions for the X-ray images and the filter coefficient is output. This filter coefficient and the original X-ray image are subjected to convolution to obtain a scattered X-ray image, and the scattered X-ray image is taken from the original X-ray image to directly obtain X-ray image.

In addition, in Japanese Patent Publication No. 3423828, a radiographic image system is described in which an intensity spread function is obtained by multiplying the intensity ratio of the dispersed light in the state where there is no subject for detection by the PSF of the dispersed light and then calculating the image of the dispersion light components. Furthermore, the intensity spread function of the scattered radiation is obtained by multiplying the PSF of the scattered radiation in a state where there is a dummy human body subject which has approximately even thickness by the intensity ratio of the scattered light obtained from image of the subject and the radiographing conditions, and the image of the scattered radiation component is computed. By subtracting the image for the diffused light component and the image for the scattered radiation component from the image of the subject, deterioration restoration of the X-ray radiographic image is performed.

However, because in performing examinations, each patient has different lesion sites and different organs for examination, as described in Japanese Patent Publication No. 2509181 and Japanese Patent Publication No. 3423828 and, even in the blurred image is restored using preset image restoration parameters that are computed based on radiographing conditions, depending on the location, blurring may not be reduced and there was a concern that accurate diagnosis was difficult.

That is to say, when the positions for examination in the thickness direction of the subject in each patient differ, the point spread function for the X-ray passed through each subject for examination is different and thus the image restoration parameters for deterioration restoration of blurred image of the subjects to be examined also differ, and in the deterioration restoration process which uses one image restoration parameter, there was a problem in that X ray radiographic image of each subject for examination could not be accurately subjected to deterioration restoration.

## SUMMARY OF THE INVENTION

An object of the present invention is to provide an image processing device and an image processing method which is capable of image restoration for obtaining high definition images in accordance with the position for examination.

(1) According to an image processing device reflecting an aspect of the present invention, the image processing device comprises: an image data input section for inputting image data obtained by using a detector to read a radiation ray applied from a radiation source and passed through a subject; a position input section for inputting a position for examination on the subject; a memory section for storing in advance image restoration parameters which correspond respectively to positions for examination on the subject; and a processing section for generating a restored image based on an image restoration parameter read from the memory section which corresponds to the position for examination on the subject that is input by the position input section and the image data that is input from the image data input section.

(2) According to another aspect of the invention, in the image processing device of Item 1 wherein, wherein the position input section is adapted to selectively input the position for examination in a direction of the radiation ray applied from the radiation source, and the memory section stores image restoration parameters based on point spread functions which correspond respectively to the positions for examination on the subject.

(3) According to still another aspect of the present invention, in the image processing device of Item 1 or Item 2, the image processing device further comprising: an image restoration parameter generating section which generates image restoration parameters based on reference image data corresponding to preset positions for examination input from the image data input section.

(4) According to an image processing method reflecting another aspect of the present invention, the method comprises: inputting image data obtained by using a detector to read radiation ray that is applied from a radiation source and passed through an subject; inputting a position for examination on the subject; reading an image restoration parameter corresponding to the position for examination on the subject that was selected in the position input step among image restoration parameters that are stored in advance corresponding to each position for examination on the object; and generating a restored image based on the image restoration parameter that has been read in the reading step and the image data that has been input at the image data input step.

(5) According to still another aspect of the present invention, in the image processing method recited in Item 4, the position input step performs selective input of the position for examination in a direction of radiation ray applied from the radiation source, and the image restoration parameter read in the reading step is based on a point spread function which corresponds to the position for examination on the object.

(6) According to yet another aspect of the present invention, in the image processing method recited in Item 4 or Item 5, the image processing method further comprises: inputting reference image data that is linked to preset positions for examination; and generating the image restoration parameters based on reference image data input in the reference image input step and links the image restoration parameters with the positions for examination and stores them.

According to the present invention, image restoration parameters corresponding to each position for examination are stored in advance in the memory section, and thus the parameters corresponding the positions for examination are read from the memory section and if restored images are generated using the image restoration parameters and the image data, even if the positions for examination are different, fine images can be restored. As a result, it becomes possible to perform diagnosis with high accuracy at any position for examination.

**BRIEF DESCRIPTION OF THE DRAWINGS**

Fig. 1 is a side view showing the schematic structure of a radiographic image radiographing system including the image processing device of this embodiment.
Fig. 2 is a block diagram showing the main control structure of the image processing device of Fig. 1.
Fig. 3 is an explanatory diagram showing the operation screen displayed in operation section included in the image processing device of Fig. 2.
Fig. 4 is an explanatory drawing showing the device structure for obtaining image data for obtaining PSF
Fig. 5 is an explanatory diagram showing the positions for examination in the chest area and the abdomen area.
Fig. 6 is graph showing a specific example of PSF.
Fig. 7 is a flowchart of an image processing method that is performed in the image processing device of Fig. 2.
Fig. 8 is an explanatory diagram showing the flow of the restoration process.
Fig. 9 is an explanatory diagram showing the principle for generating blurred images using the radiation source of the prior art.

**DESCRIPTION OF THE PREFERRED EMBODIMENT**

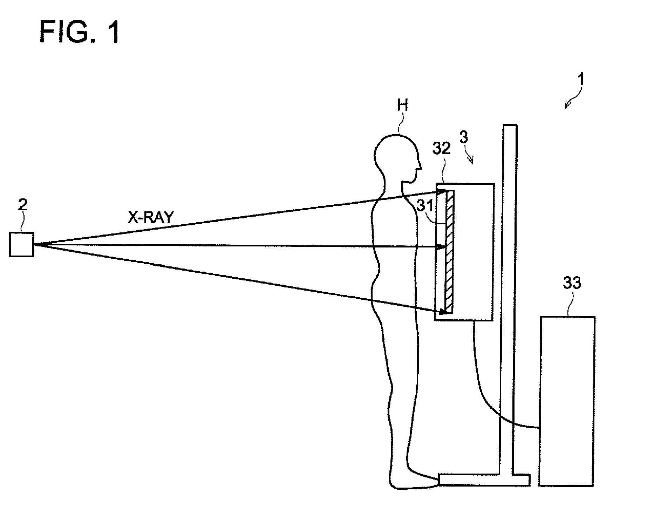

The following is a description of the image processing device of this embodiment with reference to the drawings. Fig. 1 is an explanatory diagram showing the schematic structure of a radiographic image radiographing system including the image processing device. As shown in Fig. 1, the radiographic image radiographing system 1 comprises an X-ray source 2 which irradiates X-rays as radiation and a radiographing device 3 which radiographs X-ray images for the subject H using X-rays that have been irradiated from the X-ray source 2 and passed through the subject H.

The radiographing device 3 comprises a radiographing section 32 which includes a detector 31, a main body 33 for performing radiographing control and the like.

The radiographing section 32 has a built-in detector 31 and is formed such that its height position can be adjusted to match the radiographing site.

The detector 31 detects radiated X-rays and is arranged so as to oppose the X-ray source 2. A phosphorescent plate or FPD (flat panel detector) which can absorb and store X-ray energy is used as the detector 31. For example, in the case where a phosphorescent plate is used, a reading section in which excitation light such as a laser beam or the like is irradiated on the phosphorescent plate and the phosphorescent light that is radiated from the phosphorescent plate is photoelectrically converted to image signals is provided in the radiographing section 32. The image signal (analog signal) generated by the reading section is output to the main body 33.

Meanwhile, the FDP has conversion elements arranged in a matrix which generate electric signals in accordance with the amount of X-rays input and is different from the phosphorescent plate in that electric signal are generated directly in the FDP. For example, in the case where FPD is used as the detector 31, the electric signals generated in the FPD are subjected to A/D conversion and the obtained digital image data is output to the main body section 33.

The main body section 33 is connected to the radiographing section 32 and has loaded an image processing device 10 which performs image processing for the image signals or the digital image data input from the detector 31.

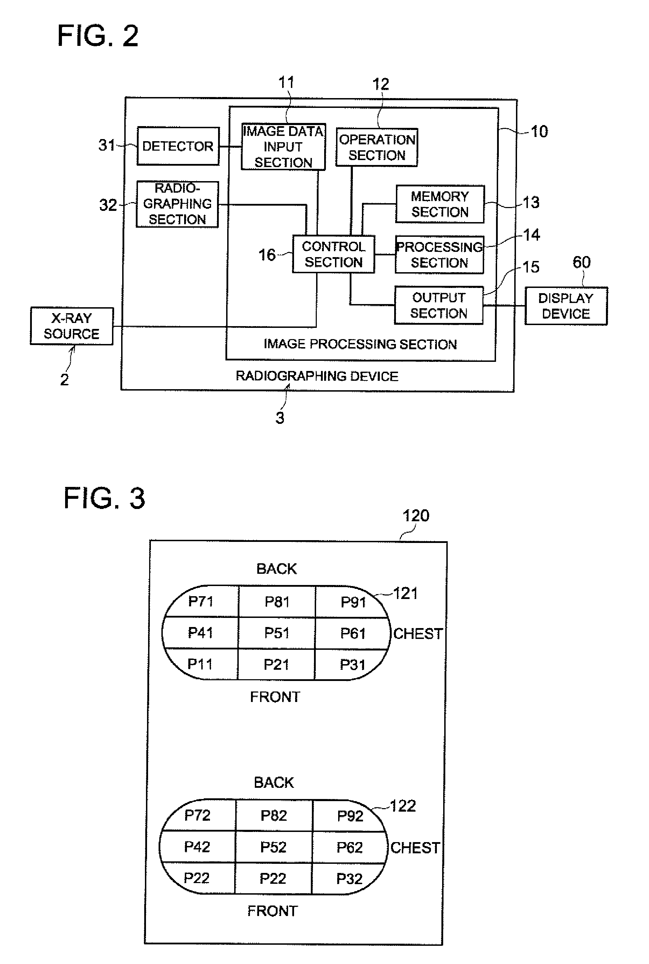

Fig. 2 is a block diagram showing the main control configuration of the image processing device 10. As shown in Fig.2, the image processing device 10 comprises: an image data input section 11 in which image data (image signal or digital image data)read at the detector 31 is input; an operation section 12 in which various commands are input; a memory section 13 which stores various control data and image data; a processing section 14 which performs image processing for the image data based on the control data in the memory section 13; an output section 15 which outputs image data that was subjected to image processing to an external display device 60; and a control section 16 which controls all these sections.

The image data input section 11 is electrically connected to the detector 31 and allows input of the image data that is read at the detector 31.

The operation section 12 is formed from a known touch panel type operation panel for example, and the radiographing start command, the restored image generate command, the radiographing conditions, the positions for examination and the like are input by operations by the user. In addition, the operation section 12 functions as the input section of the present invention which selects the position for examination of the subject H. When the position for examination of the subject H is to be selected, an operation screen with a model of the subject such as that shown in Fig. 3 for example is displayed in the operation section 12. The screen 120 is displayed so as to be divided into the chest area 121 (right surface side P11, middle surface side P21, left surface side P31, right center P41, middle center P51, left center P61, right bottom side P 71, center bottom side P81, left bottom side P91), and the abdomen area 122 (right surface side P12, center surface side P22, left surface side P32, right center P42, middle center P52, left center P62, right bottom side P 72, center bottom side P82, left bottom side P92), and selective input of the positions for examination is done by touching those positions.

In the memory section 13 the PSF (point spread function) for example is used as the image restoration parameter and it is linked with the position for examination and stored. The PSF is the function used when restoring a blurred image. The image restoration process is described hereinafter. The PSF is set for each position for examination and these PSFs are stored in advance in the memory section 13.

The processing section 14 reads the PSF corresponding to the position for examination on the subject H selected at the operation section 12 from the memory section 13 and the restored image is generated based on the PSF and the image data that was stored in the memory section 13.

The control section 16 can perform command operations for the tube voltage, X-ray radiation conditions or radiation timing for tube current X-ray in the X-ray source 2 based on the command put in the operation section 12 and in the control section 16, central control of the operation of the X-ray source 2 and the radiographing section 32 is performed in accordance with the command operations.

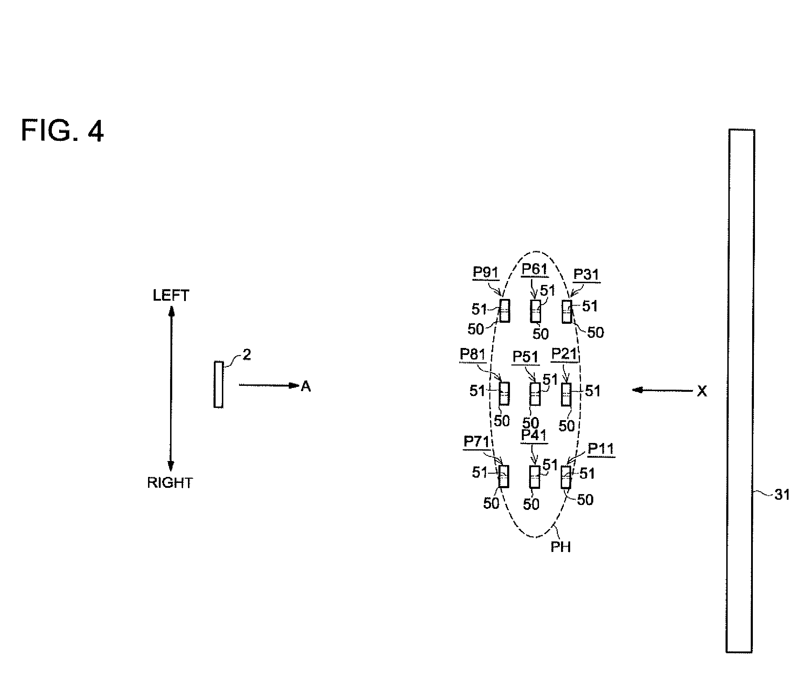

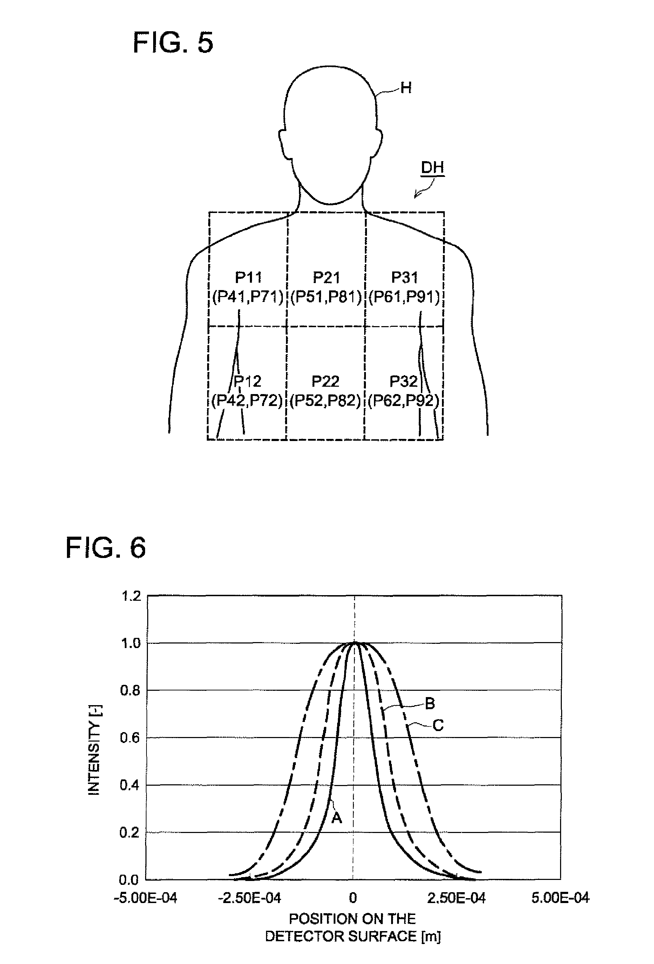

The computing process for the PSF that is stored in the memory section 13 and linked to the positions for examination that can be selectively input at the operation section 12, namely, the image restoration parameter generation process will be described next. The computing process of the PSF is performed before object radiography (for example, when installing the device, when maintenance is done, when the X-ray source or the detector 31 is replaced) and is performed for each position for examination. As shown in Fig. 4, in the computing process for PSF, the radiation source is formed of a material such as lead and the like which shields radiation, and a phantom PH having plurality of plate materials 50 in which a penetration hole 51 is formed and arranged at prescribed positions is used. The thickness of the plate material 50 is 0.01 - 0.1 mm, and the diameter of the penetration hole 51 is between a few $\mu$m and 50 $\mu$m. In the phantom PH each plate material 50 is arranged at a location corresponding to a position for examination at the time of radiographing the object. For example in Fig. 4, the case is shown in which a plate material 50 is arranged at each of the positions for examination (right surface side P11, middle surface side P21, left surface side P31, right center P41, middle center P51, left center P61, right bottom side P 71, center bottom side P81, left bottom side P91) of the chest area and abdomen area. It is to be noted that Fig. 4 is a view of phantom PH from above, while Fig. 5 is a view of phantom PH from the arrow X direction of Fig. 4, and they show the positions for examination of the chest area (right surface side P11, middle surface side P21, left surface side P31) and the abdomen area (right surface side P12, middle surface side P22, left surface side P32).

In addition, when the PSF for the positions for examination is obtained, it is preferable that the plate materials 50 are arranged so that they do not overlap in the direction of radiation from the X-ray source in order to prevent the image sharpness for the plate material from being limited due to other plate materials 50 that are not involved. That is to say, in this embodiment, the plate materials 50 corresponding to the positions for examination at the surface side, the center and the bottom side are radiographed individually for the surface side, the center and the bottom side. It is to be noted that plate material 50 may be sequentially arranged at each of the positions for examination and radiography may be performed at each.

The flow for the PSF calculation process will be described in the following. The user places the phantom PH at the same position as the position for radiographing object H in the radiographing device 3 and then inputs the PSF generation start command. When this is input, the control section 16 controls the x-ray source 2 and the radiographing section 32 and the image in the penetration hole 51 in the plate material 50 that is placed on the phantom PH is radiographed and the obtained reference image data is input to the image data input section 11 from the radiographing section 32 (reference image data input step). When the reference image data is input from the image data input section 11, the control section 16 generates luminance distribution from the image data, and a function which is similar to the linear form of the luminance distribution is formed. The function that is formed is the PSF of the positions for examination.

PSF is a spread function such as that shown in Fig. 6. Fig. 6 shows a PSF in which the amount of displacement on the image receiving surface of the detector 31 when the center position of the penetration hole 51 is shown as 0 on the horizontal axis and the maximum value for the intensity of the radiation ray that is detected in the displacement amount is shown as 1.0 on the vertical axis. In Fig. 6, A shows the PSF for the positions for examination closest to the detector 31 (60 mm from the surface of the detector 31) and C shows the PSF for the positions for examination closest to the X-ray source 2 (260 mm from the surface of the detector 31). It is to be noted that PSF shown in Fig. 6 is computed based on a focal diameter of 500 $\mu$m for the X-ray radiation source, a distance of 650 mm from the X-ray radiation source 2 to the detector 31, and reference image data at a diameter of 10 $\mu$m for the penetration holes 51.

In A of Fig. 6, there is little blurring effect caused by the focal diameter of the X-ray source 2 and the distribution of the radiation rays is narrow. On the other hand, in C of Fig. 6, there is large blurring effect caused by the focal diameter of the X-ray source 2 and the distribution of the radiation rays is wide. That is to say, in the case of the positions for radiation that are close to the detector 31, restoration parameters with little blurring effect caused by the focal diameter are used and in the case of positions that are separated from the detector 31, restoration parameters with a large blurring effect caused by the focal diameter are used and thus favorable image restoration becomes possible.

It is to be noted that in this embodiment, PSF corresponding to blurring caused by focal diameter is used, but in addition to blurring caused by focal diameter, PSF including blurring caused by scattered radiation inside the object may also be used.

It is to be noted in the case where image data of the penetration holes 51 in the multiple plate materials 50 is included, in the image data of a single radiography it is preferable that the image data is divided into single image data for each plate material 50 and penetration hole 51 and then the luminance distribution each of the images for the penetrations holes 51 is generated. The PSF that is generated are linked with the positions for examination and stored in the memory section 13. This process is repeated until the PSF of all the positions for radiation are generated.

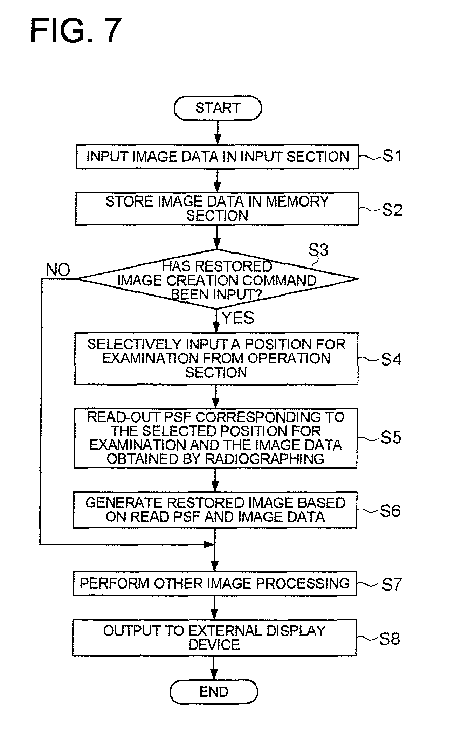

Next, the image processing method of the present invention that is performed using the radiographic image radiographing system 1 of the present invention will be described. Fig. 7 is a flowchart showing an image processing method. When the user inputs a radiography start command by operating the operation section 12, the control section 16 controls the X-ray source 2 and the radiographing section 32 and the image H is radiographed and the obtained image data is input into the image data input section 11 from the radiographing section 32 (Step S1: image data input step).

In Step S2, the control 16 stores the image data input to the image data input section 11 in the memory section 13.

In Step S3, the control section 16 determines whether the restored image creation command has been input to the image data input section 11, and if it has been input the process moves to Step S4, while if it has not been input, it moves to Step S7.

In Step S4, the positions for examination are selectively input by the operation section 12 using the operation screen 120 shown in Fig. 3, and moves to Step 5.

In Step S5, the control section 16 reads the PSF corresponding to the positions for examination selected by the operation section 12 and the image data obtained by radiographing from the memory section 13 (readings step) and moves to Step S6.

In Step S6, the control section 16 generates a restored image based on the read PSF and the image data (processing step) and moves to Step S7.

In Step S7, the control section 16 performs image processing other than restoration processing for unrestored image data or restored image data then moves to Step S8.

In Step S8, the control section 16 controls the output section 15 and outputs image data that has been subjected to image processing in Step S7 to an external device 60 and the process ends.

The restored image creation process performed in Step S6 will be described using Fig. 8. Fig. 8 is an explanatory diagram showing the flow of the restored image creation process. When the image data for the object H that was read from memory 13 is input (Step S61), the processing section 14 performs Fourier transformation of the image data (Step S62). Subsequently, the processing section 14 reads the PSF from the memory section 13(Step S63) and then the PSF that was read is subjected to Fourier transformation (Step S64). Subsequently, the processing section 14 divides the image data that was subjected to Fourier transformation in Step S62 by the PSF that was subjected to Fourier transformation in Step S64 (Step S65) and then the result of the calculation was subjected to inverse Fourier transformation (Step S66), and the restored image (image data for restored image) is generated.

It is to be noted that it is preferable to carry out the equation below based on Bayes estimation rather than simply by deconvolution. In equation (2), H is X-ray projection image, W is the restored deteriorated image and S is PSF. Also, in equation (2), a = (1, k-K+1)$_{max}$, b = (k,I)$_{min}$. K is the element number of the vector S and I is the element number of the vector W and k = {1, 2,...,K} and i = {1, 2, ...,I}. r is an integer greater than 0 which shows the repetition frequency.

[Equation 1]

$$W_{i,r+1} = W_{i,r} \sum_{k=i}^{c} \frac{S_{k-i+1} H_k}{\sum_{j=a}^{b} S_{k-j+1} W_{j,r}} \quad \cdots (1)$$

[Equation 2]

$$W_{i,1} = \sum_{k=i}^{c} \frac{S_{k-i+1} H_k}{\sum_{j=a}^{b} S_{k-j+1}} \quad \cdots (2)$$

According to the above embodiment, the PSF corresponding to each of the positions for examination are stored in advance in the memory section 13 and thus the PSF corresponding to the positions for examination are read from the memory section 13 and restored images are generated using the PSF along with the image data, even if the positions for examination are different, fine images can be restored. As a result, it becomes possible to perform diagnosis with high accuracy at any position for examination.

In addition, in the image processing device 10 of this embodiment, the position for examination can be selectively input in the direction of radiation of the radiation rays, and in particular, it becomes possible to generate fine restored images in which consideration is given to the point spread function caused by the focal diameter of the x-ray source 2.

Also, according to this embodiment, the image restoration parameters are generated based on reference image data that has been linked in advance with positions for examination and stored and thus it becomes possible to generate restored images in accordance with individual difference between the x-ray source 2 and the radiographing device 3.

It is to be noted that the present invention is, as a matter of course not limited to the above embodiment and suitable modifications are possible.

For example, in this embodiment, images in which the object is divided for each of the positions for examination are displayed at the operation section 12 and thus the example is described in which the positions for examination can be selected but the coordinates for the positions for examination may be directly input to thereby select the positions for examination. In this case the reference point for the coordinates may for example be such that the center and Z coordinates (depth direction) of the detector 31 at the X coordinate (horizontal direction) and Y coordinate (vertical direction) is the surface of the detector 31.

In addition, in this embodiment, the case where the PSF for positions for examination is generated and used in the restoration process, but the PSF is preferably generated corresponding not only to each position for examination but also to the X-ray source 2 and the detector 31. For example, if the PSF for each position for examination is generated immediately after replacement of the X-ray source 2 and the detector 31, it becomes possible to generate PSF for each position for examination corresponding to the X-ray source 2 and the detector 31 as well. As a result, PSF can be generated in which the individual difference of the X-ray source 2 and the detector 31 are taken into consideration and more favorable restoration processing becomes possible.

**Claims**

1.  An image processing device comprising:

    an image data input section for inputting image data obtained by using a detector to read a radiation ray applied from a radiation source and passed through a subject;
    a position input section for inputting a position for examination on the subject;
    a memory section for storing in advance image restoration parameters which correspond respectively to positions for examination on the subject; and
    a processing section for generating a restored image based on an image restoration parameter read from the memory section which corresponds to the position for examination on the subject that is input by the position input section and the image data that is input from the image data input section.

2.  The image processing device of Claim 1,
    wherein the position input section is adapted to selectively input the position for examination in a direction of the radiation ray applied from the radiation source, and
    the memory section stores image restoration parameters based on point spread functions which correspond respectively to the positions for examination on the subject.

3.  The image processing device of Claim 1 or Claim 2, further comprising:

    an image restoration parameter generating section which generates image restoration parameters based on reference image data corresponding to preset positions for examination input from the image data input section.

4.  An image processing method comprising:

    inputting image data obtained by using a detector to read radiation ray that is applied from a radiation source and passed through an subject;
    inputting a position for examination on the subject;
    reading an image restoration parameter corresponding to the position for examination on the subject that was selected in the position input step among image restoration parameters that are stored in advance corresponding to each position for examination on the object; and
    generating a restored image based on the image restoration parameter that has been read in the reading step and the image data that has been input at the image data input step.

5.  The image processing method of Claim 4,
    wherein the position input step performs selective input of the position for examination in a direction of radiation ray applied from the radiation source, and
    the image restoration parameter read in the reading step is based on a point spread function which corresponds to the position for examination on the object.

6.  The image processing method of Claim 4 or Claim 5, further comprising:

    inputting reference image data that is linked to preset positions for examination; and
    generating the image restoration parameters based on reference image data input in the reference image input step and links the image restoration parameters with the positions for examination and stores them.

# FIG. 1

# FIG. 2

IMAGE DATA INPUT SECTION — 11

OPERATION SECTION — 12

31 — DETECTOR

32 — RADIO-GRAPHING SECTION

MEMORY SECTION — 13

16 — CONTROL SECTION

PROCESSING SECTION — 14

OUTPUT SECTION — 15

DISPLAY DEVICE — 60

X-RAY SOURCE

2

10

IMAGE PROCESSING SECTION

RADIOGRAPHING DEVICE

3

# FIG. 3

120

BACK

| P71 | P81 | P91 |
| P41 | P51 | P61 |
| P11 | P21 | P31 |

121

CHEST

FRONT

BACK

| P72 | P82 | P92 |
| P42 | P52 | P62 |
| P22 | P22 | P32 |

122

CHEST

FRONT

# FIG. 4

LEFT

RIGHT

2

A

P91 P61 P31

51 51 51

50 50 50

P81 P51 P21

51 51 51

50 50 50

P71 P41 P11

51 51 51

50 50 50

PH

X

31

EP 1 903 787 A2

## FIG. 5

## FIG. 6

# FIG. 7

START

INPUT IMAGE DATA IN INPUT SECTION — S1

STORE IMAGE DATA IN MEMORY SECTION — S2

S3

HAS RESTORED IMAGE CREATION COMMAND BEEN INPUT?

NO

YES

SELECTIVELY INPUT A POSITION FOR EXAMINATION FROM OPERATION SECTION — S4

READ-OUT PSF CORRESPONDING TO THE SELECTED POSITION FOR EXAMINATION AND THE IMAGE DATA OBTAINED BY RADIOGRAPHING — S5

GENERATE RESTORED IMAGE BASED ON READ PSF AND IMAGE DATA — S6

PERFORM OTHER IMAGE PROCESSING — S7

OUTPUT TO EXTERNAL DISPLAY DEVICE — S8

END

## FIG. 8

```
   ┌─────────────────┐
   │  RESTORATION    │
   │    PROCESS      │
   └────────┬────────┘
            │
   ┌────────▼────────┐
   │ IMAGE DATA INPUT│──S61
   └────────┬────────┘
            │
   ┌────────▼────────┐
   │    FOURIER      │──S62
   │ TRANSFORMATION  │
   └────────┬────────┘
            │
   ┌────────▼────────┐
   │  PSF READ-OUT   │──S63
   └────────┬────────┘
            │
   ┌────────▼────────┐
   │FOURIER TRANSFORMATION│──S64
   │     OF PSF      │
   └────────┬────────┘
            │
   ┌────────▼────────┐
   │    DIVISION     │──S65
   └────────┬────────┘
            │
   ┌────────▼────────┐
   │ INVERSE FOURIER │──S66
   │ TRANSFORMATION  │
   └────────┬────────┘
            │
       ┌────▼────┐
       │   END   │
       └─────────┘
```

## FIG. 9

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2006255574 A
- JP 2509181 B
- JP 3423828 B